Europäisches Patentamt

European Patent Office

Office européen des brevets

(11) Publication number: **0 281 104 B1**

## EUROPEAN PATENT SPECIFICATION

(45) Date of publication of patent specification: **21.07.93**

(51) Int. Cl.5: **C12N 15/60**, C12N 15/68, C12N 1/20, C12P 13/22, //(C12N1/20,C12R1:19)

(21) Application number: **88103172.8**

(22) Date of filing: **02.03.88**

The file contains technical information submitted after the application was filed and not included in this specification

(54) **Novel plasmids.**

(30) Priority: **03.03.87 JP 46856/87**
**19.11.87 JP 290484/87**

(43) Date of publication of application:
**07.09.88 Bulletin 88/36**

(45) Publication of the grant of the patent:
**21.07.93 Bulletin 93/29**

(84) Designated Contracting States:
**DE FR GB NL**

(56) References cited:
**EP-A- 0 180 192        WO-A-86/05515**
**JP-A-61 239 896        JP-A-61 247 393**
**JP-A-61 247 394        US-A- 3 808 101**

**Patent Abstracts of Japan, unexamined applications, C field, vol. 9, no. 108, May 11, 1985, The Patent Office Japanese Government, p. 132 C 280**

(73) Proprietor: **RESEARCH ASSOCIATION FOR UTILIZATION OF LIGHT OIL**
**4-11, Shiba 3-chome Minato-ku**
**Tokyo(JP)**

(72) Inventor: **Shimazu, Mitsunobu**
**8-3-12, Chuo Ami-machi**
**Inashiki-gun Ibaraki-ken(JP)**
Inventor: **Terasawa, Masato**
**1-11-5-401, Chuo Ami-machi**
**Inashiki-gun Ibaraki-ken(JP)**
Inventor: **Yukawa, Hideaki**
**6-23-9, Chuo Ami-machi**
**Inashiki-gun Ibaraki-ken(JP)**

(74) Representative: **Kraus, Walter, Dr. et al**
**Patentanwälte Kraus, Weisert & Partner**
**Thomas-Wimmer-Ring 15**
**W-8000 München 22 (DE)**

## Description

This invention relates to novel plasmids comprising a DNA fragment (tna A) containing tryptophanase structural genes, a DNA fragment containing a promoter capable of permitting the structural genes to express, and regulatory genes capable of regulating the promoter. More specifically, it relates to plasmids comprising a DNA fragment containing tryptophanase structural genes, a DNA fragment containing a promoter capable of permitting the tryptophanase structural genes to express, and a DNA fragment containing a promoter and an operator for a tryprophan operon. The novel plasmids have a high copy number and excellent stability in host cells, and in cell proliferation, are exactly partitioned between daughter cells without deletion.

With regard to the stability of constructed plasmids within host cells, various genetic unstabilities of plasmids during cultivation, such as loss of plasmids from the host or the deletion of the inserted genes, have been reported, and countermeasures have been studied.

For example, a method of stabilizing the properties of a microorganism containing a plasmid was proposed in which a plasmid having inserted thereinto a chromosomal gene DNA fragment governing the property of a microorganism of the genus Escherichia to be independent from streptomycin is included into a streptomycin-dependent mutant of the genus Escherichia (U. S. Patent No. 4,371,615). This method, however, is economically disadvantageous. Moreover, since complex functions need to be incorporated in the desired plasmid, it is anticipated that the plasmid will not easily be partitioned stably between daughter cells during cell division and proliferation of the host. Industrial application of this method would, herefore, be considerably difficult.

On the other hand, the mechanism of expression of tryptophanase genes is controlled by an expression regulatory mechanism called "catabolite repression" (see Botsford, J. L. and R. D. DeMoss: J. Bac., 105, pages 303-312, 1971). When a culture medium containing glucose as a main carbon source is used in cultivating tryptophanase-containing cells, the expression of the tryptophanase genes is very strongly repressed. Accordingly, in the cultivation of E. coli containing tryptophanase, it is difficult to use a generally used culture medium containing glucose as a main carbon source.

EP-A 180192 discloses a plasmid which comprises a DNA fragment containing genes which govern the biosynthesis of tryptophane synthase, a DNA fragment derived from F-plasmid and a DNA fragment derived from a Col El-type plasmid.

WO 86/05515 discloses a method of synthesizing L-tryptophan from glycine, formaldehyde and indole using biocatalytic amounts of serine hydroxymethyltransferase, tetrahydrofolic acid and tryprophan synthetase or tryptophanase. The serine hydroxymethyltransferase and tryptophan synthetase or tryptophanase are preferably provided to the reaction medium in the form of expression products of transformed microorganisms containing expression vectors coding for serine hydroxymethyltransferase and tryptophan synthetase or tryptophanase.

The present inventors have extensively worked on the above two problems, and have now found that the stability of a plasmid containing tryptophanase structural genes can be greatly increased by introducing a certain DNA fragment derived from F-plasmid into the plasmid, and that the repression of the expression of tryptophanase structural genes by glucose can be avoided by modifying the quality of the expression regulatory genes.

More specifically, the present inventors noted that a DNA fragment containing genes which govern the partition system of F plasmid has the ability to contribute greatly to the stabilization of a plasmid, and that a Col El-type plasmid of Escherichia coli usually has a copy number of several tens per cell chromosome. On the basis of this finding, the present inventors have now succeeded in creating a novel plasmid which has a high copy number, can be partitioned stably between daughter cells and does not undergo catabolite repression by combining a DNA fragment containing genes governing the autonomous replication system of a Col El-type plasmid, a DNA fragment containing genes governing the partition system of F-plasmid, a DNA fragment containing the above tryptophanase structural genes, and a DNA fragment containing a tryptophan promoter and operator which was noted as an expression regulatory gene not undergoing catabolite repression.

According to this invention, there is provided a novel plasmid consisting essentially of

(a) a DNA fragment consisting of a tryptophanase structural gene, obtained by digesting the plasmid pMTP-1 with the restriction endonucleases BamHI and HindIII to prepare an about 3.2 kb DNA fragment containing the tryptophanase structural gene, and deleting a region extending to a Sau3AI site, said Sau3AI site being in a position separated by 1 kb from a BamHI site on said about 3.2 kb DNA fragment, thereby making a DNA fragment having a molecular size of about 2.2 kb;

(b) a DNA fragment containing a tryptophan promoter and operator which control the expression of the tryptophanase structural gene and which is derived from the plasmid pDR 720;

(c) a DNA fragment containing a gene governing the autonomous replication system and being derived from plasmid pBR 322; and

(d) a mini-F fragment having a molecular size of about 6.7 kb obtained by digesting the mini-F fragment having a molecular size of about 9.2 kb which is derived from the plasmid pMTP-1, deposited as FERM BP-1733, with the restriction endonucleases BamHI and EcoRI.

Moreover there is provided a process for producing L-tryptophan, which comprises reacting indole, pyruvic acid or its salt, and an ammonium ion in the presence of cultivated cells of E. coli K-12 strain transformed with a plasmid consisting essentially of

(a) a DNA fragment containing a tryptophanase structural gene obtained by digesting the plasmid pMTP-1 with the restriction endonucleases BamHI and HindIII to prepare an about 3.2 kb DNA fragment containing the tryptophanase structural gene, and deleting a region extending to a Sau3AI site, said Sau3AI site being in a position separated by 1 kb from a BamHI site on said about 3.2 kb DNA fragment, thereby making a DNA fragment having a molecular size of about 2.2 kb;

(b) a DNA fragment containing a tryptophan promoter and operator which control the expression of the tryptophanase structural gene and which is derived from the plasmid pDR720;

(c) a DNA fragment containing a gene governing the autonomous replication system and being derived from plasmid pBR322; and

(d) a mini-F fragment having a molecular size of about 6.7 kb obtained by digesting the mini-F fragment having a molecular size of about 9.2 kb which is derived from the plasmid pMTP-1, with the restriction endonucleases BamHI and EcoRI.

The "DNA fragment containing tryptophanase structural genes" (to be sometimes referred to as the "T fragment" hereinafter) which constitutes the plasmid of this invention denotes a DNA fragment containing genes which govern the biosynthesis of tryptophanase, an enzyme having a role of decomposing tryptophan into indole, pyruvic acid and ammonia.

The "DNA fragment containing a tryptophan promoter and operator which control the expression of the tryptophanase structural genes" (to be sometimes referred to as "P fragment") is a DNA fragment containing a promoter and an operator fraction of a tryptophan operon, and means a gene which governs the control of the expression of a gene joined downstream. The P fragment is derived from plasmid pDR720 (made by Pharmacia Co.).

A fragment containing both the T fragment and the P fragment, which is derived from E. coli, may be conveniently used in practical applications. There is no particular limitation on microorganisms which become sources of supply of the T fragment and the P fragment. Advantageously, however, E. coli ATCC 23282, E. coli ATCC 23437, and E. coli ATCC 27325 are used.

The method of preparing the DNA fragment containing tryptophanase structural genes (T fragment), which can be used in this invention, is described in detail in Example 1, (A), (C) and (D), given hereinafter. The basic procedure of obtaining the T fragment is to extract chromosomal DNA having a tryptophanase operon from E. coli, and digest the chromosomal DNA or plasmid pMTP-1 with restriction endonucleases BamHI and HindIII to isolate a tryptophanase operon DNA fragment and a region extending to a Sau3AI site, said site being in a tryptophanase operon DNA fragment is deleted and thereby the intended T fragment can be obtained.

The method of preparing the P fragment is described in detail in Example 1, (F) given hereinbelow. The basic procedure is to cut out a tryptophan operon from a chromosomal DNA of E. coli by using restriction endonucleases SalI and XhoI as in the procedure of obtaining the P fragment. The resulting DNA fragment contains the tryptophan promoter and operator.

One characteristic feature of this invention is that the T fragment and the P fragment are combined with the DNA fragment containing genes which are derived from F-plasmid and govern its partition system. The F-plasmid is a known plasmid having a molecular size of $62 \times 10^6$ daltons (94.5 kb) with the structure shown in the genetic map and the physical map by EcoRI given at page 64 of R. A. Skurray, H. Nagaishi and A. J. Clark: Proc. Natl. Acad. Sci., U. S. A., vol. 73, 1976. The F-plasmid exists in enteric bacteria such as E. coli usually in a copy number of 1 to 2 per cell chromosome. This plasmid has a mechanism whereby it is accurately transmitted to daughter cells after cell division (the proliferation accurately in accord with the pace of proliferation of a host in spite of a low copy number level as in this case is called the "stringent" control of proliferation). It has already been found that such a function of stringently controlling proliferation in the F-plasmid is carried on a fragment, called mini-F, which has a molecular size of $6 \times 10^6$ daltons (9.2 kb) and can replicate autonomously ["Molecular & General Genetics", 196, 185-193 (1984)]. It is also known that the mini-F can be cut out from the F-plasmid by restriction endonuclease EcoRI.

The present invention utilizes the partition system of the mini-F. Thus, the "DNA fragment containing genes which are derived from the F plasmid and govern its partition system" (to be sometimes abbreviated as the "F fragment") denotes a DNA fragment having a mechanism of accurately transmitting the F-plasmid to daughter cells. A typical example of such F fragment is a mini-F fragment having a molecular size of about 9.2 kb.

A DNA fragment having a size of about 6.7 kb cut out with BamHI and EcoRI in the about 9.2 kb mini-F fragment cut out with EcoRI is used in this invention. These DNA fragments have both a cell division coupling mechanism and a uniform partitioning mechanism which are the two plasmid stabilizing mechanism of the mini-F [see T. Ogura, S. Hiraga: Proc. Natl. Acad. Sci., U. S. A., vol. 80, 1983, page 4784].

The "DNA fragment containing genes which are derived from a Col El-type plasmid and which govern its autonomous replication" (to be sometimes abbreviated as "S fragment") to be used in combination with the T, P and F fragments in this invention denotes a DNA fragment which has a copy number of 20 to 30 per cell chromosome and contains genes governing the autonomous replication of the Col El-type plasmid. This S fragment is an S fragment derived from the plasmid pBR322 and having a size of about 4.3 kb.

The plasmids provided by this invention comprise the T fragment, P fragment, F fragment and S fragment as essential components, and may further include DNA fragments bearing other genetic information, for example a DNA fragment containing an ampicillin-resistance gene which is an antibiotic resistance marker, and a DNA fragment containing a kanamycin-resistance gene.

Typical examples of the plasmid of this invention are plasmids consisting essentially of the T, P, F and S fragments and having a molecular size of about 8.5 megadaltons (about 13.0 kb) and about 8.2 megadaltons (about 12.7 kb) which are respectively named "plasmid pMTP-2", and "plasmid pMTP-3 and pMTP-3R" by the present inventors.

In the present application, the molecular sizes of plasmids are determined by the agarose gel electrophoretic method.

These plasmids pMTP-2, pMTP-3 and pMTP-3R will be described in detail hereinafter.

The sensitivities (the number of recognition or cleavage sites) of these plasmids to the following restriction endonucleases, and the molecular weights (megadaltons) of fragments cleaved with these restriction endonucleases are shown below.

## Plasmid pMTP-2

| Restriction endonuclease | Number of recognition sites | Molecular weight of the fragment (megadaltons *) |
|---|---|---|
| HindIII | 1 | about 8.5 (13.0) |
| XhoI | 1 | about 8.5 (13.0) |
| EcoRI | 2 | about 8.43 (12.9), about 0.07 (0.1) |
| PvuI | 2 | about 3.2 (4.9), about 5.3 (8.1) |
| BglII | 2 | about 1.3 (2.0), about 7.2 (11) |

(*): The parenthesized figures in the above and subsequent tables are the corresponding lengths (kb).

4

| Plasmid pMTP-3 | | |
|---|---|---|
| Restriction endonuclease | Number of recognition sites | Molecular weight of the fragment (megadaltons *) |
| EcoRI | 2 | 1.3 (2.0), 6.9 (10.7) |
| BglII | 2 | 1.3 (2.0), 6.9 (10.7) |
| XhoI | 1 | 8.2 (12.7) |
| BamHI | 2 | 2.6 (4.1), 5.6 (8.6) |
| SalI | 3 | 1.3 (2.0), 2.4 (3.8), 4.5 (6.9) |

| Plasmid pMTP-3R | | |
|---|---|---|
| Restriction endonuclease | Number of recognition sites | Molecular weight of the fragment (megadaltons *) |
| EcoRI | 2 | 1.3 (2.0), 6.9 (10.7) |
| BglII | 2 | 1.3 (2.0), 6.9 (10.7) |
| XhoI | 1 | 8.2 (12.7) |
| BamHI | 2 | 3.8 (6.8), 4.4 (5.9) |
| SalI | 3 | 0.1 (0.2), 3.6 (5.6), 4.5 (6.9) |

Plasmids pMTP-3 and pMTP-3R are products of molecular biological modification of plasmid pMTP-2 for enhanced productivity of tryptophan. Specifically, plasmids pMTP-3 and pMTP-3R are obtained by substituting the about 6.6 kb DNA fragment cut out from the mini-F fragment with restriction endonucleases BamHI and SalI and the about 6.7kb DNA fragment cut out from mini-F fragment with restriction endonucleases BamHI and EcoRI respectively for the DNA fragment derived from F-plasmid and governing its partition system. The modified plasmids have a markedly increased ability with cell proliferation and thus increase the total activity of tryptophan synthesis.

The plasmids pMTP-2, pMTP-3 and pMTP-3R having the properties described above can be produced, for example, by the following procedures.

Construction of plasmid pMTP-2

pMTP-1 containing T and F fragments is isolated from cultivated cells of E. coli K12 YK3002, (FERM BP-1733) by a conventional method [T. Maniatis, E. F. Fritsch, Sambrook: "Molecular Cloning" (1982), pages 86 to 94]. A DNA fragment containing the T fragment is prepared by cleavage with HindIII and BamHI, and a DNA fragment containing the F fragment, by cleavage with BamHI and SalI.

The P fragment may be prepared by digestion of commercial plasmid pDR720 (a product of Pharmacia Co.) with EcoRI. Alternatively, a plasmid containing the P fragment is isolated in a customary manner from cultivated cells of E. coli K12 YK2004, (FERM BP-1732) and a DNA fragment containing the P fragment may be prepared by cleavage with SalI and XhoI. Plasmid pMTP-2 can be obtained by ligating the resulting DNA fragments containing T, F and P fragments, respectively, with plasmid pBR322 by DNA ligase derived from T4 phage.

The method of constructing plasmid pMTP-2 will be described in more detail hereinbelow in Example 1.

Construction of plasmids pMTP-3 and pMTP-3R

As in the construction of plasmid pMTP-2, pMTP-1 containing T and F fragments is isolated from cultivated cells of E. coli K12 YK3002, (FERM BP-1733) by a conventional method [T. Maniatis, E. F. Fritsch, Sambrook: "Molecular Cloning" (1982), pages 86 to 94]. A DNA fragment containing the T fragment is prepared by cleavage with HindIII and BamHI, and a DNA fragment containing the F fragment, by cleavage with BamHI and EcoRI.

The P fragment can be prepared as in the construction of plasmid pMTP-2.

The resulting DNA fragments containing the T, F and P fragments are ligated with plasmid pBR322 by a DNA ligase derived from T4 phage. As a result, plasmids pMTP-3 and pMTP-3R can be obtained.

5

The method of constructing plasmids pMTP-3 and pMTP-3R will be described in more detail in Example 5.

The plasmids of this invention constructed as above have a high copy number, are hardly deleted when partitioned between daughter cells in the cell division of the host, and do not undergo catabolite repression in the expression of the tryptophanase genes.

Accordingly, the plasmids of this invention are much expected to find industrial utility in the production of tryptophan or its derivatives. In the production of tryptophan, the host is transformed with the plasmids of the invention. E. coli is preferred as a host organism that can be utilized in the transformation.

The plasmids of the invention can be introduced into the host organism by methods known per se, for example, the method described in M. Mandel, A. Higa: J. Mol. Biol., 53, 159 (1970).

By cultivating the transformed host microorganism by a method known per se, tryptophanase can be produced and accumulated sufficiently in the cells.

Tryptophanase decomposes tryptophan to indole, pyruvic acid and ammonia. It is also known from, for example, Agricultural and Biological Chemistry, vol. 36, No. 13, pages 2523-2528 (1972) and US-A-3,808,101 that tryptophanase can be utilized in enzyme reactions for producing L-tryptophan or L-tryptophan derivatives from indoles such as indole, 5-hydroxyindole, 5-aminoindole and 5-methylindole, at least one of organic acids such as pyruvic acid, oxzaloacetic acid, malic acid, fumaric acid, glyoxylic acid and lactic acid, and an ammonium ion, or from the above indoles and at least one of amino acids such as cysteine, cystine, S-methylcystine and serine.

The microorganism cells cultivated may be directly utilized in the enzymatic reaction of producing L-tryptophan. They may, however, be used in the form of a crushed product obtained by crushing the cells by, for example, sonication, an extract obtained by extracting the crushed product with water or the like, or a partially purified product obtained by treating the extract with ammonium sulfate, etc. to precipitate the enzyme component. As required, the cells and their treated products may be immobilized prior to use.

The cultivated cells are sometimes used after they are stored in the frozen state at below 0 °C. In this case, the tryptophanase-producing cells stored in the frozen cells at below 0 °C are thawed at 60 °C or below within three hours at the longest before they are submitted to the reaction and thereafter used in the reaction within 5 hours at the longest. By so doing, L-tryptophan can be produced with a high efficiency without a decrease in enzyme activity.

The reaction of indole, pyruvic acid or its salt and an ammonium ion in the presence of the cells or the treated product thereof may be carried out, for example, in a solvent such as 0.1M phosphate buffer (pH 7.5 - 10.0) or water (pH 7.5 to 10.0) at a temperature of about 20 to about 50 °C, preferably about 30 to about 40 °C, for a period of usually about 10 to about 72 hours, as in an ordinary enzyme reaction.

The amounts of indole, pyruvic acid or its salt and an ammonium ion used in the reaction are not particularly restricted if they are used in concentrations which do not inhibit the enzyme reaction. Generally, they are suitably used respectively in a concentration of 0.1 to 20 % (wt/vol). The amount of the cells or the treated product thereof is neither restricted in particular. Generally, the cells or the treated products thereof are used in a concentration of 1 to 10 % (wt/vol).

The cultivation of the transformed host organism can be carried out generally in synthetic or natural media normally used although the selection of a suitable medium may vary depending upon the type of the host microorganism. Carbon sources that can be used may include various carbohydrates such as glucose, glycerol, fructose, sucrose and maltose. Nitrogen sources that can be used may include natural organic nitrogen sources such as tryptone, yeast extract, corn steep liquor and casein hydrolyzate. Many of the natural organic nitrogen sources can also serve as carbon sources. It is possible to induce tryptophanase activity by adding indoleacrylic acid to the culture medium.

The cultivation can be carried out under aerobic conditions by shaking culture or aeration-agitation submerged culture. The cultivation temperature is generally 20 to 50 °C, and the pH of the culture medium is desirably maintained at a neutral pH or a slightly alkaline pH. The cultivation period is usually 1 to 5 days.

Isolation and purification of L-tryptophan formed in the reaction mixture obtained by reacting indole, pyruvic acid or its salt and an ammonium ion in the presence of the cells obtained by the above cultivation or the treated products thereof may be carried out by known methods such as adsorption and desorption treatment using ion-exchange resins and charcoal.

The host microorganism transformed with the plasmids of this invention can also be utilized in the production of L-tryptophan by a fermentation method. Specifically, by cultivating the host microorganism transformed with the plasmids of this invention in a culture medium containing indole, L-tryptophan is produced and accumulated in the culture medium. L-tryptophan can thus be produced by recovering it.

The following Examples specifically illustrate the present invention.

6

EXAMPLE 1

Construction of plasmid pMTP-2

(A) Preparation of plasmid pMTP-1

One hundred milliliters of L-medium (10 g of tryptone, 5 g of yeast extract, 1 g of glucose, 5 g of NaCl, 1 liter of distilled water, pH 7.2) was put in a 500 ml Erlenmeyer flask, and sterilized at 120 °C for 15 minutes.

Ampicillin was added to this medium so that its final concentration became 50 $\mu$g/ml. E. coli K-12 YK3002 (FERM BP-1733) was inoculated in the medium, and cultivated at 37 °C for 15 hours. Two milliliters of the culture broth was inoculated in 100 ml of a fresh supply of the above medium, and again cultivated at 37 °c for 4 hours.

After the cultivation, the entire culture broth was centrifuged (8000G, 15 minutes, 4 °C) to harvest the cells. Plasmid pMTP-1 was extracted from the cells by the alkali-SDS method [see T. Maniatis, E. F. Fritsch, J. Sambrook: "Molecular Cloning" (1982), pages 90-91].

(B) Preparation of mini-F fragment and plasmid pBR322 mini-F (BamHI/SalI fragment):-

Plasmid pMTP-1 prepared in (A) (25 $\mu$g) was digested with 5 units each of BamHI and SalI at 37 °C for 1 hour. After the reaction, the digestion products were subjected to agarose gel electrophoresis, and by utilizing the difference in molecular weight, 2 $\mu$g of a DNA fragment having a molecular size of about 6.6 kb was prepared.

One micrograms of pBR322 (a product of Takara Shuzo Co., Ltd.) was digested with 5 units each of BamHI and SalI at 37 °C for 1 hour, and then by maintaining the digestion product at 65 °C for 10 minutes, BamHI and SalI were inactivated. The inactivated solution was supplemented with 50 mM Tris buffer (pk 7.6), 10 mM $MgCl_2$, 10 mM dithiothreitol, 1 mM ATP and 1 unit of T4 ligase as final concentrations, and then maintained at 16 °C for 15 hours. Using this solution, E. coli K12 strain (tryptophanase-deficient and tryptophan required mutant) was transformed in a customary manner. The transformants were plated on L-medium containing ampicillin in a final concentration of 50 $\mu$g/ml, and cultivated at 37 °C for 2 days. The plasmid was isolated from the grown cells by the alkali SDS method and digested with BamHI (5 units) and SalI (5 units). The molecular weights of the resulting fragments were measured by agarose gel electrophoresis. There were obtained five suitable plasmids [pBR322 mini-F(BamHI/SalI fragments)] in which the about 6.6 kb fraction of the mini-F fragment contained in plasmid pMTP-1 was inserted into the BamHI and SalI sites.

(C) Preparation of a DNA fragment containing a tryptophanase operon:-

The plasmid pMTP-1 (25 $\mu$g) prepared in (A) above was digested with BamHI and HindIII (5 units each) at 37 °C for 1 hour. After the reaction, the digestion products were subjected to agarose gel electrophoresis, and by utilizing the difference in molecular weight, 1.8 $\mu$g of a DNA fragment having a molecular size of about 3.2 kb was prepared.

(D) Construction of plasmid pUC18tnaA:-

One microgram of the BamHI/HindIII DNA fragment prepared in (C) above was digested with 5 units of SphI at 37 °C for 1 hour. Furthermore, 0.5 $\mu$g of plasmid pUC18 (a product of Takara Shuzo Co., Ltd.) was digested with 5 units each of HindIII and SphI at 37 °C for 1 hour. The digestion products were mixed, and maintained at 65 °C for 10 minutes to inactivate the restriction endonucleases. The inactivated solution was supplemented with 50 mM Tris buffer (pH 7.6), 10 mM $MgCl_2$, 10 mM dithiothreitol, 1mM ATP and 1 unit of T4 ligase as final concentrations, and maintained at 16 °C for 15 hours.

Using the resulting solution, E. coli JM109 (a product of Takara Shuzo Co., Ltd.) was transformed in a customary manner [see Methods in Enzymology, 101, 20 - 78 (1988)]. The transformants (JM109 pUC18 SphI/HindIII fragment) were cultivated in L-medium containing ampicillin in a concentration of 50 $\mu$g/ml, and the plasmid was isolated by the alkali SDS method from the culture broth.

One microgram of the plasmid was digested with 5 units each of BamHI and SphI at 37 °C for 1 hour. Furthermore, 1 $\mu$g of the BamHI/HindIII DNA fragment prepared in (C) was digested with 5 units each of Sau3AI and SphI at 37 °C for 1 hour. The digestion products were maintained at 65 °C for 10 minutes to inactivate the restriction endonucleases. The inactivated solution was supplemented with 50 mM Tris buffer (pH 7.6), 10 mM MgCl$_2$, 10 mM dithiothreitol, 1 mM ATP and 1 unit of T4 ligase as final concentrations, and then maintained at 16 °C for 15 hours. Using this solution, E. coli K12 strain (tryptophanase-deficient and tryptophan required mutant) in a customary manner. The transformants were plated on a selective medium (7 g of K$_2$HPO$_4$, 2 g of KH$_2$PO$_4$, 1 g of (NH$_4$)$_2$SO$_4$, 0.1 g of MgSO$_4$·7H$_2$O, 5 g of casamino acids, 50 mg of adenine hydrochloride, 2 g of glycerol, 30 mg of ampicillin, 20 g of agar, 1 liter of deionized water) and cultivated at 37 °C for 24 hours. The plasmid was isolated from the grown cells by the alkali SDS method, and digested with 5 units each of EcoRI and HindIII. The molecular weights of the digestion products were measured by agarose gel electrophoresis. As a result, insertion of a DNA fragment having a size of about 2.2 kb was determined.

(E) Construction of pBR322tnaA

By extraction from the transformants obtained in (D) by the alkali SDS method, 25 μg of plasmid pUC18tnaA was obtained. This plasmid was digested with 5 units each of EcoRI and HindIII at 37 °C for 1 hour. After the reaction, the digestion product was subjected to agarose gel electrophoresis and by utilizing the difference in molecular weight, 2 μg of a DNA fragment having a molecular size of about 2.2 kb was obtained.

Then, 1 μg of the plasmid pBR322 mini-F BamHI/SalI fragment obtained in (B) above was digested with EcoRI and HindIII (5 units) at 37 °C for 1 hour. The two digestion products were mixed, and maintained at 65 °C for 10 minutes to inactivate the endonucleases. The inactivated solution was supplemented with 50 mM Tris buffer (pH 7.6), 10 mM MgCl$_2$, 10 mM dithiothreitol, 1 mM ATP and 1 unit of T4 ligase as final concentrations, and maintained at 16 °C for 15 hours. Using this solution, E. coli K-12 strain (tryptophanase-deficient and tryptophan required mutant) was transformed in a customary manner. The transformants were placed on L-medium containing ampicillin in a final concentration of 50 μg/ml. The plasmid was extracted from the grown cells by the alkali SDS method, and digested with 5 units each of EcoRI and HindIII. The molecular weights of the digestion products were measured by agarose gel electrophoresis. As a result, there was obtained a plasmid resulting from insertion of a DNA fragment having a size of about 2.2 kb in the EcoRI and HindIII sites of the plasmid pBR322 mini-F BamHI/SalI fragment.

This plasmid contained a part (BamHI/SalI fragment) of mini-F as a plasmid stabilizing factor and tryptophanase structural gene (tnaA) in plasmid pBR322.

(F) Ligation of pBR322tnaA with tryptophan promoter and operator:-

By the same method as in (A) above, E. coli K-12 YK2004 (FERM BP-1732) was cultivated, and the plasmid pMTY-2 contained in it was prepared by the alkali SDS method.

Plasmid pMTY-2 (25 μg) was digested with 5 units each of XhoI and SalI at 37 °C for 1 hour, and by utilizing the difference in molecular weight in agarose electrophoresis, 2 μg of a DNA fragment having a molecular size of about 7 kb was obtained. This DNA fragment (2 μg) was digested partially with 5 units of AluI and 2 units of TaqI at 37 °C for 1 hour, and the termini of the DNA fragment were smoothened by reaction with one unit of Bal31 at 30 °C for 3 minutes. By maintaining the digestion products at 65 °C for 10 minutes, the restriction endonucleases were deactivated. Then, a synthetic oligonucleotide (having an EcoRI site at both ends) was added and by a conventional method (Molecular Cloning, p. 396, Cold Spring Harbor Laboratory), joined to the above DNA fragment.

One microgram of the plasmid prepared in (E) was digested with 3 units of EcoRI at 37 °C for 1 hour. The two products were mixed, and supplemented with 50 mM Tris buffer (pH 7.6), 10 mM MgCl$_2$, 10 mM dithiothreitol, 1 mM ATP and 1 unit of T4 ligase as final concentrations, and the solution was maintained at 16 °C for 15 hours. Using this solution, E. coli K-12 strain (tryptophanasedeficient and tryptophan required mutant) was transformed in a customary manner. The transformants were plated on the aforesaid selective medium and cultivated at 37 °C for 2 days. The plasmid was extracted from the grown cells by the alkali SDS method. The plasmid was digested with 5 units of EcoRI, and the molecular weight of the resulting fragment was measured by using agarose gel electrophoresis. Insertion of a DNA fragment having a size of about 100 bp in the EcoRI site of the plasmid was determined.

These transformants were cultivated in the following media A, B, C and D, and the tryptophan activity was examined in accordance with Referential Example 1 below, and the results are shown in Table 1.

8

| Composition | Culture medium | | | |
|---|---|---|---|---|
| | A | B | C | D |
| Trypton | 10 g | 10 g | 10 g | 10 g |
| Yeast extract | 5 g | 5 g | 5 g | 5 g |
| NaCl | 5 g | 5 g | 5 g | 5 g |
| Glucose (*) | 1 g | - | 1 g | - |
| Indoleacrylic acid (**) | 200 mg | 200 mg | - | - |
| Deionized water | 1 ℓ | 1 ℓ | 1 ℓ | 1 ℓ |

(*):  separately sterilized

(**): separately added

## Table 1

| Culture medium | Relative activity |
|---|---|
| A | 100 |
| B | 100 |
| C | 80 |
| D | 80 |

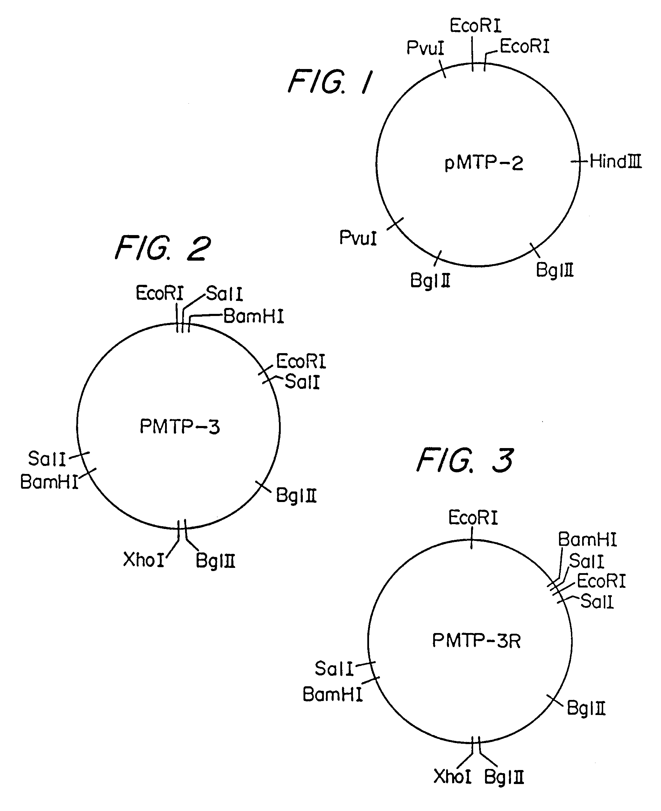

The results given in Table 1 led to the determination that the tryptophanase structural genes of this plasmid expresses under the control of the promoter-operator of the tryptophan operon. Thus, the plasmid pMTP-2 was constructed. The restriction endonuclease cleavage map of this plasmid is shown in Figure 1.

REFERENTIAL EXAMPLE 1

Measurement of tryptophanase activity:-

One hundred milliliters of each of the media A, B, C and D was put in a 500 ml Erlenmeyer flask, and sterilized at 120 °C for 15 minutes. The transformants were inoculated in the medium and cultivated at 37 °C for 1 day under shaking. Then, 2 ml of the culture broth was inoculated in 100 ml of the same medium prepared as above and cultivated under shaking at 37 °C for 8 hours. The culture broth was centrifuged to harvest the cells. The cells were washed with 100 mM Tris buffer (pH 8.0) and again centrifuged. The wet cells (200 mg) were suspended in 1 ml of 100 mM Tris buffer (pH 8.0), and ultrasonicated. After the treatment, the disrupted cells were diluted suitably with 100 mM Tris buffer (pH 8.0) and added to a reaction solution containing 100 $\mu$moles of Tris buffer (pH 8.0), 100 $\mu$moles of KCl, 10 $\mu$moles of L-tryptophan and 0.03 $\mu$mole of pyridoxal-5-phosphoric acid per ml and reacted at 37 °C for 15 minutes. By a conventional method [0. H. Smith and C. Yanofasky: "Methods in Enzymology", Academic, New York (1962), vol. 5, pages 794-806], the formed indole was quantitatively determined.

EXAMPLE 2

Introduction of plasmid pMTP-2 into E. coli

One hundred milliliters of L-medium (10 g of trypton, 5 g of yeast extract, 5 g of NaCl, 1 g of glucose, 1 liter of distilled water, pH 7.2) was put in a 500 ml Erlenmeyer flask, and sterilized at 120 °C for 15 minutes. A mutant (tryptophan required, adenine required and tryptophanase-deficient) obtained by treating E. coli K12 ATCC 27325 by a known method (a Japanese-language publication, "Experimental Agrochemistry", Volume B, 3rd edition, pages 226-230, edited by Tokyo University, Department of Agricultural Science, Agrochemistry Section and published by Asakura Shoten on May 25, 1978) was inoculated in the above medium, and cultivated at 37 °C for 15 hours. Two milliliters of the culture medium was collected, inoculated freshly in 100 ml of the above medium, and again cultivated at 37 °C for 2 hours. After the cultivation, 30 ml of the cultivation product was aseptically centrifuged (8000 G, 5 minutes, 4 °C) to harvest

EP 0 281 104 B1

the cells. The harvested cells were suspended in 30 ml of a sterilized 100 mM $MgCl_2$ solution, and centrifuged (8000G, 5 minutes, 4 °C), and re-suspended in 10 ml of sterilized 100 mM $CaCl_2$ cooled at 0 °C. The suspension was cooled for 1 hour in ice.

After the cooling, 0.5 $\mu$g of plasmid pMTP-2 was added to 100 microliters of this suspension, and the mixture was cooled in ice for 30 minutes. Then, it was heated at 42 °C for 2 minutes and plated on a selective medium [7 g of $K_2HPO_4$, 2 g of $KH_2PO_4$, 1 g of $(NH_4)_2SO_4$, 0.1 g of $MgSO_4$-$H_2O$, 5 g of casamino acids, 50 mg of adenine hydrochloride, 2 g of glucose, 20 mg of ampicillin, 20 g of agar and 1 liter of deionized water] and cultivated at 37 °C for 24 hours to obtain grown cells. The cells transformed with plasmid pMTP-2 were named YK3004.

The transformants E. coli K-12 YK3004 harboring the plasmid pMTP-2 were deposited in Fermentation Research Institute, Agency of Ministry of International Trade and Industry, at 1-3, Higashi, 1-chome, Tsukuba-shi

Ibaraki-ken, Japan on February 26, 1987 (deposit number FERM BP-1735).

EXAMPLE 3

Stability of the transformants

One hundred milliliters of the aforesaid selective medium was put in a 500 ml. Erlenmeyer flask, and sterilized at 120 °C for 15 minutes. The transformants obtained in Example 2 were inoculated in the selective medium and cultivated under shaking at 37 °C for 24 hours. Then, 100 ml of L-medium prepared in the same way was put in a 500 ml Erlenmeyer flask and sterilized at 120 °C for 15 minutes. The cells were inoculated at a density of 50 cells per ml, cultivated at 37 °C for 24 hours under shaking, and then centrifuged. The harvested cells were washed, and a fixed amount of the cells was plated on a plate medium obtained by adding ampicillin to the L-medium at a rate of 50 $\mu$g/ml, or L-medium not containing ampicillin, and cultivated at 37 °C for 1 day. After the cultivation, the number of colonies grown was counted.

It was consequently determined that the colonies grown on the ampicillin-containing medium were the same in number as those grown on the ampicillin-free medium; and that the colonies grown on the L-medium all grow on the selective medium used in Example 2. Thus, the high stability of the plasmid was determined.

EXAMPLE 4

Production of L-tryptophan

Fifty milliliters of a culture medium of the following composition was put in a 500 ml Erlenmeyer flask, and sterilized at 120 °C for 15 minutes. The transformants, E. coli K-12 YR3004 (FERM BP-1735) obtained in Example 2 were inoculated in the above culture medium, and cultivated under shaking at 37 °C for 1 day. Two milliliters of the culture broth was inoculated in 100 ml of the same medium except that it contained indoleacrylic acid in a concentration of 100 $\mu$g/ml, and cultivated under shaking at 37 °C for 12 hours.

| Composition of the culture medium | |
|---|---|
| $KH_2PO_4$ | 2 g |
| $K_2HPO_4$ | 7 g |
| $(NH_4)_2SO_4$ | 1 g |
| $MgSO_4$-$7H_2O$ | 100 mg |
| Adenine hydrochloride | 50 mg |
| Yeast extract | 1 g |
| Trypton | 1 g |
| Glucose | 1 g |
| Indoleacrylic acid | 100 mg |
| deionized water | 1000 ml |
| pH | 7.2 |

10

The cells were harvested using a centrifugal separator. The harvested cells were suspended in 50 ml of 100 mM Tris buffer (pH 8.5) containing 1.5 g of indole, 1.5 g of sodium pyruvate, 1.5 g of ammonium acetate, 0.5 mg of pyridoxal-5-phosphoric acid and 5 g of "Triton X-100", and reacted at 37 °C for 2 hours with shaking. After the reaction, the reaction product was diluted to 10-fold with water, and centrifuged. The supernatant was analyzed for the formed L-tryptophan by high-performance liquid chromatography. L-tryptophan was found to form in an amount of 2.1 mg/ml.

After the reaction, 500 ml of the diluted solution was passed through a column of ammonia-type strong acid ion-exchange resin (Diaion SK-1B, a product of Mitsubishi Chemical Co., Ltd.) to adsorb L-tryptophan on it. The column was eluted with an alkali solution and the eluate was concentrated to precipitate L-tryptophan as crude crystals. The crude crystals were washed with acetone and dried to give 0.7 g of L-tryptophan as crystals.

EXAMPLE 5

Construction of plasmids pMTP-3 and pMTP-3R

(A) Preparation of plasmid pMTP-1

One hundred milliliters of L-medium (10 g of trypton, 5 g of yeast extract, 1 g of glucose, 5 g of NaCl, 1 liter of distilled water, pH 7.2) was put in a 500 ml Erlenmeyer flask, and sterilized at 120 °C for 15 minutes.

Ampicillin was added to the medium so that its final concentration reached 50 $\mu$g/ml. E. coli K-12 YK3002 (FERM BP-1733) was inoculated in the medium and cultivated at 37 °C for 15 hours. Two milliliters of the culture broth was taken and inoculated in 100 ml of the same medium, and again cultivated at 37 °C for 4 hours.

After the cultivation, the entire culture broth was centrifuged (8000G, 15 minutes, 4 °C) to collect the cells. The plasmid was extracted from the cells by the alkali SDS method. Thus, pMTP-1 was obtained.

(B) Preparation of mini-F fraction and construction of plasmid pBR322 mini-F (BamHI/EcoRI fragment):-

The plasmid pMTP-1 constructed in (A) above (25 $\mu$g) was digested with 5 units each of BamHI and EcoRI at 37 °C for 1 hour. The digestion product was subjected to agarose gel electrophoresis, and by utilizing the difference in molecular size, 2 $\mu$g of a DNA fragment having a molecular size of about 6.7 kb was prepared.

One microgram of pBR322 (a product of Takara Shuzo Co., Ltd.) was digested with 5 units each of BamHI and EcoRI a 37 °C for 1 hour, and the restriction endonucleases were inactivated by maintaining the digestion product at 65 °C for 10 minutes. The inactivated solution was supplemented with 50 mM Tris buffer (pH 7.6), 10 mM $MgCl_2$, 10 mM dithiothreitol, 1 mM ATP and 1 unit of T4 ligase as final concentrations, and maintained at 16 °C for 15 hours. Using this solution, E. coli K12 strain (tryptophanase-deficient and tryptophan required mutant) was transformed in a customary manner. The transformants were plated on L-medium containing ampicillin in a final concentration of 50 $\mu$g/ml and cultivated at 37 °C for 2 hours. The plasmid was extracted from the grown cells by the alkali SDS method, and digested with 5 units each of BamHI and EcoRI. The molecular weights of the digestion products were measured by agarose gel electrophoresis. As a result, five plasmids [pBR322 mini-F (BamHI/EcoRI fragment)] were obtained in which the about 6.7 kb fraction of the mini-F fragment contained in plasmid pMTP-1 was inserted into the BamHI and EcoRI sites.

(C) Preparation of a DNA fragment containing a tryptophanase operon:-

The plasmid pMTP-1 prepared in (A) above (25 $\mu$g) was digested with 5 units each of BamHI and HindIII at 37 °C for 1 hour. After the reaction, the digestion products were subjected to agarose electrophoresis, and by utilizing the difference in molecular weight, 1.8 $\mu$g of a DNA having a molecular size of about 3.2 kb was prepared.

(D) Construction of plasmid pUC18tnaA[+]

One microgram of the BamHI/HindIII DNA fragment prepared in (C) was digested with 5 units of SphI at 37 °C for 1 hour. Separately, 0.5 $\mu$g of plasmid pUC18 (a product of Takara Shuzo Co., Ltd.) was digested

EP 0 281 104 B1

with 5 units each of HindIII and SphI at 37 °C for 1 hour. The digestion products were mixed and the endonucleases were deactivated by maintaining the mixture at 65 °C for 10 minutes. The inactivated solution was supplemented with 50 mM Tris buffer (pH 7.6), 10 mM $MgCl_2$, 10 mM dithiothreitol, 1 mM ATP and 1 unit of T4 ligase as final concentrations, and maintained at 16 °C for 15 hours.

Using this solution, E. coli JM109 (a product of Takara Shuzo Co., Ltd.) was transformed in a customary manner [see Methods in Enzymology, 101, 20 - 78 (1983)] to obtain transformants. The transformants (JM109 pUC18 SphI/HindIII fragment) were cultivated in L-medium containing ampicillin in a concentration of 50 $\mu$g/ml, and the plasmid was isolated by the alkali SDS method.

The plasmid (1 $\mu$g) was digested with 5 units each of BamHI and SphI at 37 °C for 1 hour. Separately, 1 $\mu$g of the BamHI/HindIII DNA fragment prepared in (C) above was digested with 5 units each of Sau3AI and SphI at 37 °C for 1 hour. These digestion products were mixed, and the restriction endonucleases were deactivated by maintaining the mixture at 65 °C for 10 minutes. The inactivated solution was supplemented with 50 mM Tris buffer (pH 7.6), 10 mM $MgCl_2$, 10 mM dithiothreitol, 1 mM ATP and 1 unit of T4 ligase as final concentrations, and then maintained at 16 °C for 15 hours. Using the resulting solution, E. coli K12 strain (tryptophanase-deficient and tryptophan required mutant) was transformed in a customary manner. The transformants were plated on a selective medium (7 g of $K_2HPO_4$, 2 g of $KH_2PO_4$, 1 g of $(NH_4)_2SO_4$, 0.1 g of $MgSO_4 \cdot 7H_2O$, 5 g of casamino acids, 50 mg of adenine hydrochloride, 2 g of glycerol, 20 mg of ampicillin, 20 g of agar, 1 liter of deionized water), and cultivated at 37 °C for 24 hours. The plasmid was isolated from the grown cells by the alkali SDS method (pUC18tnaA$^+$), and digested with 5 units each of EcoRI and HindIII. The molecular weights of the digestion products were measured by agarose gel electrophoresis. Insertion of a DNA fragment having a molecular size of about 2.2 kb was determined.

(E) Construction of plasmid pDR720tnaA$^+$

Plasmid pUC18tnaA$^+$ (25 $\mu$g) was isolated by the alkali SDS method from the transformants obtained in (D) above, and digested with 5 units of AvaI at 37 °C for 1 hour. After the reaction, the digestion product was subjected to agarose gel electrophoresis, and by utilizing the difference in molecular weight, 2 $\mu$g of a DNA fragment having a molecular size of about 1.9 kb was obtained.

The plasmid pDR720 (a product of Pharmacia Co.) was digested with 5 units of AvaI at 37 °C for 1 hour, and the restriction endonuclease was deactivated by maintaining the digestion prodcut at 65 °C for 10 minutes.

The above digestion products were mixed, and supplemented with 50 mM Tris buffer (pH 7.6), 10 mM $MgCl_2$, 10 mM dithiothreitol, 1 mM ATP and 1 unit of T4 ligase as final concentrations, and maintained at 16 °C for 15 hours.

Using the resulting solution, E. coli K12 strain (tryptophanase-deficient and tryptophan required mutant) was transformed in a customary manner. The transformants were plated on a selective medium (7 g of $K_2HPO_4$, 2 g of $KH_2PO_4$, 1 g of $(NH_4)_2SO_4$, 0.1 g of $MgSO_4 \cdot 7H_2O$, 5 g of casamino acids, 50 mg of adenine hydrochloride, 2 g of glycerol, 20 mg of ampicillin, 20 g of agar, 1 liter of deionized water), and cultivated at 37 °C for 24 hours. The plasmid was isolated by the alkali SDS method from the grown cells, (pDR720tnaA$^+$) and digested with 5 units of EcoRI. The molecular weight of the digestion product was measured by agarose gel electrophoresis. As a result, insertion of a DNA fragment having a molecular size of about 2.0 kb was determined.

(F) Construction of pMTP-3 and pMTP-3R

pDR720tnaA$^+$ (25 $\mu$g) was isolated by the alkali SDS method from the transformants obtained in (E) above, and digested with 5 units of EcoRI at 37 °C for 1 hour. The digestion product was subjected to agarose gel electrophoresis, and by utilizing the difference in molecular weight, 2 $\mu$g of a DNA fragment having a molecular weight of about 2.0 kb was obtained.

The plasmid pBR322 mini-F BamHI/EcoRI fragment (1 $\mu$g) obtained in (B) above was digested with 5 units of EcoRI at 37 °C for 1 hour.

The above digestion products were mixed, and by maintaining the mixture at 65 °C for 10 minutes, EcoRI was inactivated. The inactivated solution was supplemented with 50 mM Tris buffer (pH 7.6), 10 mM $MgCl_2$, 10 mM dithiothreitol, 1 mM ATP and 1 unit of T4 ligase as final concentrations, and maintained at 16 °C for 15 hours.

Using the resulting solution, E. coli K-12 strain (tryptophanase-deficient and tryptophan required mutant) was transformed in a customary manner. The transformants were plated on a selective medium (7 g of $K_2HPO_4$, 2 g of $KH_2PO_4$, 1 g of $(NH_4)_2SO_4$, 0.1 g of $MgSO_4 \cdot 7H_2O$, 5 g of casamino acids, 50 mg of adenine hydrochloride, 2 g of glycerol, 20 mg of ampicillin, 20 g of agar, 1 liter of deionized water), and cultivated at 37 °C for 24 hours. The plasmid was isolated by the alkali SDS method from the grown cells, and digested with 5 units of EcoRI. The molecular weight of the digestion product was measured by agarose gel electrophoresis. As a result, insertion of a DNA fragment having a molecular size of about 2.0 kb was determined.

The plasmid was further digested with 5 units of BamHI, and the molecular weight of the digestion product was measured by agarose gel electrophoresis. There were obtained three plasmids in which the directions of insertion of the about 2.0 kb DNA fragment at the EcoRI site were different. The transformants harboring the plasmids with different insertion directions were each cultivated in the culture media A, B, C and D described in Example 1, and the tryptophanase activity was measured as in Referential Example 1. The results are shown in Table 2.

Table 2

| Medium | Transformants containing pMTP-3 | Transformants containing pMTP-3R |
|---|---|---|
| A | 100 | 100 |
| B | 100 | 100 |
| C | 80 | 80 |
| D | 80 | 80 |

The results given in Table 2 led to the determination that the tryptophanase structural genes of this plasmid expresses under the control of the promoter-operator of the tryptophan operon. Thus, the plasmid pMTP-3 and pMTP-3R were constructed. The restriction endonuclease cleavage maps of these plasmids are shown in Figures 2 and 3.

EXAMPLE 6

Introduction of plasmids pMTP-3 and pMTP-3R into E. coli K-12 strain

One hundred milliliters of L-medium (10 g of trypton, 5 g of yeast extract, 5 g of NaCl, 1 g of glucose, 1 liter of deionized water, pH 7.2) was put in a 500 ml Erlenmeyer flask, and sterilized at 120 °C for 15 minutes. A mutant (tryptophan required, adenine required and tryptophanase-deficient) obtained by treating E. coli K-12 ATCC 27325 by a known method (a Japanese-language publication, "Experimental Agrochemistry", Volume B, 3rd edition, pages 226-230, edited by Tokyo University, Department of Agricultural Science, Agrochemistry Section and published by Asakura Shoten on May 25, 1978) was inoculated in the above medium, and cultivated at 37 °C for 15 hours. Two milliliters of the culture medium was collected, inoculated freshly in 100 ml of the above medium, and again cultivated at 37 °C for 2 hours. After the cultivation, 30 ml of the cultivation product was aseptically centrifuged (8000 G, 5 minutes, 4 °C) to harvest the cells. The harvested cells were suspended in 30 ml of a sterilized 100 mM $MgCl_2$ solution, and centrifuged (8000G, 5 minutes, 4 °C), and re-suspended in 10 ml of sterilized 100 mM $CaCl_2$ cooled at 0 °C. The suspension was cooled for 1 hour in ice.

After cooling, 0.5 $\mu$g of plasmid pMTP-3 or pMTP-3R was added to 100 microliters of this suspension, and the mixture was cooled in ice for 30 minutes. Then, it was heated at 42 °C for 2 minutes and plated on a selective medium [7 g of $K_2HPO_4$, 2 g of $KH_2PO_4$, 1 g of $(NH_4)_2SO_4$, 0.1 g of $MgSO_4$-$H_2O$, 5 g of casamino acids, 50 mg of adenine hydrochloride, 2 g of glucose, 20 mg of ampicillin, 20 g of agar and 1 liter of deionized water] an cultivated at 37 °C for 24 hours to obtain grown cells. The cells transformed with plasmid pMTP-3 were named YK3005, and the cells transformed with plasmid pMTP-3R was named YK3006.

The transformants E. coli K-12 YR3005 harboring the plasmid pMTP-3 were deposited in Fermentation Research Institute, Agency of Ministry of International Trade and Industry, at 1-3 Higashi, 1-chome Tsukuba-shi, Ibaraki-ken, Japan on September 26, 1987 (deposit number FERM BP-1736).

The transformants E. coli K-12 YR3006 harboring the plasmid pMTP-3R were deposited in Fermentation Research Institute, Agency of Ministry of International Trade and Industry, at 1-3, Higashi, 1-chome Tsukuba-shi, Ibaraki-ken, Japan on September 26, 1987 (deposit number FERM BP-1737).

EXAMPLE 7

Stability of the transformants

One hundred milliliters of the aforesaid selective medium was put in a 500 ml. Erlenmeyer flask, and sterilized at 120 °C for 15 minutes. The transformants obtained in Example 2 were inoculated in the selective medium and cultivated under shaking at 37 °C for 24 hours. Then, 100 ml of L-medium prepared in the same way was put in a 500 ml Erlenmeyer flask and sterilized at 120 °C for 15 minutes. The cells were inoculated at a density of 50 cells per ml, cultivated at 37 °C for 24 hours under shaking, and then centrifuged. The collected cells were washed, and a fixed amount of the cells was plated on a plate medium obtained by adding ampicillin to the L-medium at a rate of 50 μg/ml, or L-medium not containing ampicillin, and cultivated at 37 °C for 1 day. After the cultivation, the number of colonies grown was counted.

It was consequently determined that the colonies grown on the ampicillin-containing medium were the same in number as those grown on the ampicillin-free medium; and that the colonies grown on the L-medium all grow on the selective medium used in Example 2. Thus, the high stability of the plasmids was determined.

EXAMPLE 8

Production of L-tryptophan

Fifty milliliters of the same culture medium as used in Example 4 was put in a 500 ml Erlenmeyer flask, and sterilized at 120 °C for 15 minutes. The transformants, E. coli K-12 YK3005 (FERM BP-1736) and YK3006 (FERM BP-1737), obtained in Example 6 and the transformants E. coli K-12 YK3004 (FERM BP-1735) were each inoculated in the above culture medium, and cultivated under shaking at 37 °C for 1 day. Two milliliters of the culture broth was inoculated in 100 ml of the same medium except that it contained indoleacrylic acid in a concentration of 100 μg/ml, and cultivated under shaking at 37 °C for 12 hours.

The cells were harvested by using a centrifugal separator, and the amounts of the harvested cells was measured. All the harvested cells were suspended in 50 ml of 100 mM Tris buffer (pH 8.5) containing 1.5 g of indole, 1.5 g of sodium pyruvate, 1.5 g of ammonium acetate, 0.5 mg of pyridoxalphosphoric acid and 5 g of "Triton X-100", and reacted at 37 °C for 1 hour with shaking. After the reaction, the reaction mixture was diluted to 10-fold with water and centrifuged. The supernatant obtained was analyzed for the formed L-tryptophan by high-performance liquid chromatography. The results are shown in Table 3.

Table 3

| Transformant | Relative amount of the cells yielded | L-tryptophan yielded (mg/ml). |
|---|---|---|
| YK3005 | 100 | 1.6 |
| YK3006 | 100 | 1.5 |
| YK3004 | 60 | 1.0 |

The cultivation product of YK3005 was passed through a column of ammonia-type strong acid-type ion-exchange resin (Diaion SK-1B, a product of Mitsubishi Chemical Co., Ltd.) to adsorb L-tryptophan on it. The column was eluted with an alkali solution, and the eluate was concentrated to precipitate crude crystals of L-tryptophan. The crude crystals were washed with acetone and dried to obtain 0.5 g of L-tryptophan crystals.

**Claims**

1.   A plasmid consisting essentially of
      (a) a DNA fragment consisting of a tryptophanase structural gene, obtained by digesting the plasmid pMTP-1 with the restriction endonucleases BamHI and HindIII to prepare an about 3.2 kb DNA fragment containing the tryptophanase structural gene, and deleting a region extending to a Sau3AI site, said Sau3AI site being in a position separated by 1 kb from a BamHI site on said about 3.2 kb DNA fragment, thereby making a DNA fragment having a molecular size of about 2.2 kb;
      (b) a DNA fragment containing a tryptophan promoter and operator which control the expression of the tryptophanase structural gene and which is derived from the plasmid pDR 720;

14

(c) a DNA fragment containing a gene governing the autonomous replication system and being derived from plasmid pBR 322; and

(d) a mini-F fragment having a molecular size of about 6.7 kb obtained by digesting the mini-F fragment having a molecular size of about 9.2 kb which is derived from the plasmid pMTP-1, deposited as FERM BP-1733, with the restriction endonucleases BamHI and EcoRI.

2. The plasmid of claim 1 which is plasmid pMTP-3, deposited as FERM BP-1736, having a molecular weight of about 8.2 megadaltons and 2, 2, 1, 2 and 3 sites of cleavage with restriction endonucleases EcoRI, BglII, XhoI, BamHI and SalI respectively, wherein the XhoI fragment has a molecular weight of about 8.2 megadaltons, the EcoRI and BglII fragments have a molecular weight of about 1.3 and about 6.9 megadaltons, the BamHI fragments have a molecular weight of about 2.6 and about 5.6 megadaltons, the SalI fragments have a molecular weight of about 1.3, about 2.4 and about 4.5 megadaltons.

3. The plasmid of claim 1 which is plasmid pMTP-3R, deposited as FERM BP-1737, having a molecular weight of about 8.2 megadaltons and 2, 2, 1, 2 and 3 sites of cleavage with restriction endonucleases EcoRI, BglII, XhoI, BamHI and SalI respectively, wherein the EcoRI and BglII fragments have a molecular weight of about 1.3 and about 6.9 megadaltons, the XhoI fragment has a molecular weight of about 8.2 megadaltons, the BamHI fragments have a molecular weight of about 3.8 and about 4.4 megadaltons, and the SalI fragments have a molecular weight of about 0.1, about 3.6 and about 4.5 megadaltons.

4. A microorganism of E. coli K-12 strain which is transformed with the plasmid of claims 1, 2 or 3.

5. The microorganism of claim 4 which is E. coli K-12 YK3005 (FERM BP-1736) or E. coli K-12 YK3006 (FERM BP-1737).

6. A process for producing L-tryptophan, which comprises reacting indole, pyruvic acid or its salt, and an ammonium ion in the presence of cultivated cells of E. coli K-12 strain transformed with a plasmid consisting essentially of

(a) a DNA fragment containing a tryptophanase structural gene obtained by digesting the plasmid pMTP-1 with the restriction endonucleases BamHI and HindIII to prepare an about 3.2 kb DNA fragment containing the tryptophanase structural gene, and deleting a region extending to a Sau3AI site, said Sau3AI site being in a position separated by 1 kb from a BamHI site on said about 3.2 kb DNA fragment, thereby making a DNA fragment having a molecular size of about 2.2 kb;

(b) a DNA fragment containing a tryptophan promoter and operator which control the expression of the tryptophanase structural gene and which is derived from the plasmid pDR720;

(c) a DNA fragment containing a gene governing the autonomous replication system and being derived from plasmid pBR322; and

(d) a mini-F fragment having a molecular size of about 6.7 kb obtained by digesting the mini-F fragment having a molecular size of about 9.2 kb which is derived from the plasmid pMTP-1, deposited as FERM BP-1733, with the restriction endonucleases BamHI and EcoRI.

7. The process for producing L-tryptophan of claim 6 wherein the microorganism is Escherichia coli K-12 YK3005 (FERM BP-1736) or Escherichia coli K-12 YK3006 (FERM BP-1737).

8. A process for producing L-tryptophan, which comprises reacting indole, pyruvic acid or its salt, and an ammonium ion in the presence of cultivated cells obtained by cultivating the microorganism of claim 4 or in the presence of a crushed product, a partially purified product or an immobilized product of said cultivated cells.

## Patentansprüche

1. Plasmid bestehend im wesentlichen aus

(a) einem DNA-Fragment, bestehend aus einem Tryptophanase-Strukturgen, erhalten durch Abbau des Plasmids pMTP-1 mit den Restriktionsendonukleasen BamHI und HindIII unter Herstellung eines etwa 3,2 kb langen DNA-Fragments, enthaltend das Tryptophanase-Strukturgen, und Deletion einer Region, die sich bis zu einer Sau3AI-Stelle erstreckt, wobei die Sau3AI-Stelle in einer Position ist, die 1 kb von einer BamHI-Stelle auf dem etwa 3,2 kb langen DNA-Fragment getrennt ist, wodurch ein DNA-Fragment mit einer Molekülgröße von etwa 2,2 kb hergestellt wird;

(b) einem DNA-Fragment, enthaltend einen Tryptophan-Promotor und Operator, das die Expression des Tryptophanase-Strukturgens kontrolliert und das sich von dem Plasmid pDR 720 ableitet;

(c) einem DNA-Fragment, enthaltend ein Gen, das das autonome Replikationssystem steuert und sich von dem Plasmid pBR 322 ableitet; und

(d) einem mini-F-Fragment mit einer Molekülgröße von etwa 6,7 kb, erhalten durch Abbau des mini-F-Fragments mit einer Molekülgröße von etwa 9,2 kb, das von dem Plasmid pMTP-1, hinterlegt als FERM BP-1733, mit den Restriktionsendonukleasen BamHI und EcoRI abgeleitet ist.

2. Plasmid nach Anspruch 1, dadurch gekennzeichnet, daß es Plasmid pMTP-3, hinterlegt als FERM BP-1736, ist, ein Molekulargewicht von etwa 8,2 Megadalton besitzt und 2, 2, 1, 2 und 3 Spaltstellen für die Restriktionsendonukleasen EcoRI, BglII, XhoI, BamHI bzw. SalI besitzt, worin das XhoI-Fragment ein Molekulargewicht von etwa 8,2 Megadalton besitzt, die EcoRI- und BhIII-Fragmente ein Molekulargewicht von etwa 1,3 und etwa 6,9 Megadalton besitzen, die BamHI-Fragmente ein Molekulargewicht von etwa 2,6 und etwa 5,6 Megadalton besitzen, die SalI-Fragmente ein Molekulargewicht von etwa 1,3, etwa 2,4 und etwa 4,5 Megadalton besitzen.

3. Plasmid nach Anspruch 1, dadurch gekennzeichnet, daß es Plasmid pMTP-3R, hinterlegt als FERM BP-1737 ist, ein Molekulargewicht von etwa 8,2 Megadalton besitzt und 2, 2, 1, 2 und 3 Spaltstellen für die Restriktionsendonukleasen EcoRI, BglII, XhoI, BamHI bzw. SalI besitzt, worin die EcoRI- und BglII-Fragmente ein Molekulargewicht von etwa 1,3 und etwa 6,9 Megadalton besitzen, das XhoI-Fragment ein Molekulargewicht von etwa 8,2 Megadalton besitzt, die BamHI-Fragmente ein Molekulargewicht von etwa 3,8 und etwa 4,4 Megadalton besitzen und die SalI-Fragmente ein Molekulargewicht von etwa 0,1, etwa 3,6 und etwa 4,5 Megadalton besitzen.

4. Mikroorganismus des Stammes E. coli K-12, der mit dem Plasmid nach den Ansprüchen 1, 2 oder 3 transformiert wurde.

5. Mikroorganismus nach Anspruch 4, dadurch gekennzeichnet, daß er E. coli K-12 YK3005 (FERM BP-1736) oder E. coli K-12 YK3006 (FERM BP-1737) ist.

6. Verfahren zur Erzeugung von L-Tryptophan, dadurch gekennzeichnet, daß man Indol, Brenztraubensäure oder ihr Salz und ein Ammoniumion in Gegenwart von gezüchteten Zellen des Stammes E. coli K-12, die mit einem Plasmid, bestehend im wesentlichen aus

(a) einem DNA-Fragment, enthaltend ein Tryptophanase-Strukturgen, erhalten durch Abbau des Plasmids pMTP-1 mit den Restriktionsendonukleasen BamHI und HindIII unter Herstellung eines etwa 3,2 kb langen DNA-Fragments, enthaltend das Tryptophanase-Strukturgen und Deletion einer Region, die sich bis zu einer Sau3AI-Stelle erstreckt, wobei die Sau3AI-Stelle in einer Position ist, die 1 kb von einer BamHI-Stelle auf dem etwa 3,2 kb langen DNA-Fragment getrennt ist, wodurch ein DNA-Fragment mit einer Molekülgroße von etwa 2,2 kb hergestellt wird;

(b) einem DNA-Fragment, enthaltend einen Tryptophan-Promotor und-Operator, das die Expression des Tryptophanase-Strukturgens kontrolliert und das sich von dem Plasmid pDR 720 ableitet;

(c) einem DNA-Fragment, enthaltend ein Gen, das das autonome Replikationssystem steuert und sich von dem Plasmid pBR 322 ableitet; und

(d) einem mini-F-Fragment mit einer Molekülgröße von etwa 6,7 kb, erhalten durch Abbau des mini-F-Fragments mit einer Molekülgröße von etwa 9,2 kb, das von dem Plasmid pMTP-1, hinterlegt als FERM BP-1733, mit den Restriktionsendonukleasen BamHI und EcoRI abgeleitet ist, transformiert wurde, umsetzt.

7. Verfahren zur Erzeugung von L-Tryptophan nach Anspruch 6, dadurch gekennzeichnet, daß der Mikroorganismus Escherichia coli K-12 YK3005 (FERM BP-1736) oder Escherichia coli K-12 YK3006 (FERM BP-1737) ist.

8. Verfahren zur Erzeugung von L-Tryptophan, dadurch gekennzeichnet, daß man Indol, Brenztraubensäure oder ihr Salz und ein Ammoniumion in Gegenwart von gezüchteten Zellen, erhalten durch Züchten des Mikroorganismus nach Anspruch 4, oder in Gegenwart eines zerkleinerten Produkts, eines teilgereinigten Produkts oder eines immobilisierten Produkts der gezüchteten Zellen umsetzt.

**Revendications**

1. Plasmide constitué essentiellement par

   (a) un fragment d'ADN constitué par un gène structural de tryptophanase obtenu par digestion du plasmide pMTP-1 par les endonucléases de restriction BamHI et HindIII qui donne un fragment d'ADN d'environ 3,2 kb contenant le gène structural de la tryptophanase, et délétion d'une région qui s'étend jusqu'à un site Sau3AI, ledit site Sau3AI étant situé à une distance de 1 kb d'un site BamHI sur ledit fragment d'ADN d'environ 3,2 kb, ce qui donne un fragment d'ADN ayant une taille moléculaire d'environ 2,2 kb;

   (b) un fragment d'ADN contenant un promoteur et un opérateur tryptophane qui contrôlent l'expression du gène structural de la tryptophanase et qui est dérivé du plasmide pDR 720;

   (c) un fragment d'ADN contenant un gène régissant le système de réplication autonome et dérivé du plasmide pBR 322;

   (d) un fragment mini-F ayant une taille moléculaire d'environ 6,7 kb obtenu par digestion du fragment mini-F ayant une taille moléculaire d'environ 9,2 kb, dérivé du plasmide pMTP-1, déposé sous le numéro FERM BP-1733, par les endonucléases de restriction BamHI et EcoRI.

2. Plasmide selon la revendication 1 qui est le plasmide pMTP-3, déposé sous le numéro FERM BP-1736, ayant une masse moléculaire d'environ 8,2 mégadaltons et respectivement 2, 2, 1, 2 et 3 sites de coupure avec les endonucléases de restriction EcoRI, BglII, XhoI, BamHI et SalI, où le fragment XhoI a une masse moléculaire d'environ 8,2 mégadaltons les fragments EcoRI et BglII ont une masse moléculaire d'environ 1,3 et environ 6,9 mégadaltons, les fragments BamHI ont une masse moléculaire d'environ 2,6 et environ 5,6 mégadaltons, les fragments SalI ont une masse moléculaire d'environ 1,3, environ 2,4 et environ 4,5 mégadaltons.

3. Plasmide selon la revendication 1 qui est le plasmide pMTP-3R, déposé sous le numéro FERM BP-1737, ayant une masse moléculaire d'environ 8,2 mégadaltons et respectivement 2, 2, 1, 2 et 3 sites de coupure avec les endonucléases de restriction EcoRI, BglII, XhoI, BamHI et SalI, où les fragments EcoRI et BglII ont une masse moléculaire d'environ 1,3 et environ 6,9 mégadaltons, le fragment XhoI a une masse moléculaire d'environ 8,2 mégadaltons, les fragments BamHI ont une masse moléculaire d'environ 3,8 et environ 4,4 mégadaltons, et les fragments SalI ont une masse moléculaire d'environ 0,1, environ 3,6 et environ 4,5 mégadaltons.

4. Microorganisme de la souche E. coli K-12 qui est transformé avec le plasmide selon les revendications 1, 2 ou 3.

5. Microorganisme selon la revendication 4 qui est E. coli K-12 YK3005 (FERM BP-1736) ou E. coli K-12 YK3006 (FERM BP-1737).

6. Procédé de production de L-tryptophane qui comprend la réaction d'indole, d'acide pyruvique ou d'un de ses sels, et d'un ion ammonium en présence de cellules cultivées d'une souche d'E. coli K-12 transformée avec un plasmide constitué essentiellement par

   (a) un fragment d'ADN constitué par un gène structural de tryptophanase obtenu par digestion du plasmide pMTP-1 par les endonucléases de restriction BamHI et HindIII qui donne un fragment d'ADN d'environ 3,2 kb contenant le gène structural de la tryptophanase, et délétion d'une région qui s'étend jusqu'à un site Sau3AI, ledit site Sau3AI étant situé à une distance de 1 kb d'un site BamHI sur ledit fragment d'ADN d'environ 3,2 kb, ce qui donne un fragment d'ADN ayant une taille moléculaire d'environ 2,2 kb;

   (b) un fragment d'ADN contenant un promoteur et un opérateur tryptophane qui contrôlent l'expression du gène structural de la tryptophanase et qui est dérivé du plasmide pDR 720;

   (c) un fragment d'ADN contenant un gène régissant le système de réplication autonome et dérivé du plasmide pBR 322;

   (d) un fragment mini-F ayant une taille moléculaire d'environ 6,7 kb obtenu par digestion du fragment mini-F ayant une taille moléculaire d'environ 9,2 kb, dérivé du plasmide pMTP-1, déposé sous le numéro FERM BP-1733, par les endonucléases de restriction BamHI et EcoRI.

7. Procédé de production de L-tryptophane selon la revendication 6, dans lequel le microorganisme est Escherichia coli K-12 YK3005 (FERM BP-1736) ou Escherichia coli K-12 YK3006 (FERM BP-1737).

8. Procédé de production de L-tryptophane, qui comprend la réaction d'indole, d'acide pyruvique ou d'un de ses sels, et d'un ion ammonium en présence de cellules cultivées obtenues par culture du microorganisme selon la revendication 4 ou en présence d'un produit broyé, d'un produit partiellement purifié ou d'un produit immobilisé desdites cellules cultivées.

FIG. 1

pMTP-2

FIG. 2

PMTP-3

FIG. 3

PMTP-3R